# EUROPEAN PATENT APPLICATION

(11) **EP 2 105 103 A1**
(43) Date of publication of application: **30.09.2009**
(21) Application number: 09004487.6
(22) Date of filing: 27.03.2009
(51) Int. Cl.: A61B 18/14, A61B 17/00, A61M 1/00

(54) **Bioadhesive applicator for ENT surgery**

(30) Priority: 28.03.2008 US 40353 P; 10.02.2009 US 368434
(71) Applicant: Tyco Healthcare Group, LP, North Haven CT 06473 (US)
(72) Inventor: Dycus, Sean, T., Broomfield CO 80020 (US)
(74) Representative: HOFFMANN EITLE

(57) **Abstract**

The present disclosure relates to an electrosurgical suction coagulator and includes a housing having an elongated electrode and a fluid applicator. The elongated electrode includes distal and proximal ends and is adapted to connect to an energy source, for example, an electrosurgical generator. The proximal end of the elongated electrode is configured to operably couple to a distal end of the housing. Further, the distal end of the elongated electrode is configured to apply energy to tissue. The elongated electrode also includes a lumen defined therethrough, that is operably coupled to a vacuum source. The fluid applicator assembly is operably coupled to the elongated electrode and includes a container defining a reservoir. The reservoir is configured to contain a bioadhesive therein. The selectively dispensable from the container to deliver the bioadhesive to a surgical site.

## Description

### CROSS-REFERENCE TO RELATED APPLICATIONS

This application claims the benefit of priority to U.S. Provisional Application Serial No. 61/040,353 entitled "BIOADHESIVE APPLICATOR FOR ENT SURGERY" filed March 28, 2008 by Sean T. Dycus, which is incorporated by reference herein.

### BACKGROUND

### Technical Field

The present invention relates generally to electrosurgical coagulators and, more particularly, to an electrosurgical suction coagulator having a medicinal fluid applicator assembly.

### Description of Related Art

Electrosurgical suction coagulators that coagulate tissue have been available for some time. Generally, these devices include a conductive suction tube having an insulating coating over all but a most distal portion of the tube so that the distal portion forms a generally annular ablating electrode. A suction source is attached to a proximal portion of the tube for evacuating excess fluid and debris from the surgical site through the distal end of the tube.

The coagulation of bleeding blood vessels and tissue using electrically conductive suction tubes is a technique, which has been widely used in the medical field, particularly electrosurgery, for some time. Typically, a combination electrocautery and suction device is employed during ear, nose and throat (ENT) surgery whenever excessive blood and tissue debris must be removed from the bleeding site in order to facilitate hemostasis of any bleeding vessels. After removing or treating tissue or organs, such as tonsils or adenoids, a medicinal fluid (e.g., bioadhesive fluid) may be applied to facilitate healing.

Typically, the user must stop the coagulation and/or the suction procedure, remove the coagulation instrument, insert a bioadhesive applicator and release the bioadhesive material to or into the tissue. If the user decides to perform an additional coagulation and/or suction treatment, the coagulation instrument must be redeployed to the tissue site, thus making it more time consuming for the user and patient and possibly complicating the surgical procedure.

### SUMMARY

The present disclosure relates to an electrosurgical suction coagulator and includes a housing having an elongated electrode and a fluid applicator. The elongated electrode includes distal and proximal ends and is adapted to connect to an energy source, for example, an electrosurgical generator. The proximal end of the elongated electrode is configured to operably couple to a distal end of the housing. Further, the distal end of the elongated electrode is configured to apply energy to tissue. The elongated electrode also includes a lumen defined therethrough, that is operably coupled to a vacuum source. The fluid applicator assembly is operably coupled to the elongated electrode and includes a container defining a reservoir configured to hold a bioadhesive therein. The bioadhesive is selectively dispensable from the container to deliver the bioadhesive to a surgical site.

In embodiments, the fluid applicator assembly includes a seal or valve to selectively regulate the flow of bioadhesive to the surgical site. Further, the fluid applicator assembly may include a container or bladder that is compressible to expel the bioadhesive from the container to the surgical site. The fluid applicator assembly may include an actuator that is moveable from a first position, wherein the bioadhesive is maintained in the container, to a subsequent position to incrementally dispense the bioadhesive to the surgical site. Such devices may include a plunger or syringe-like assembly.

In other embodiments, the fluid applicator assembly may include an actuator that is motorized. For example, the actuator may include a motorized screw-like element that forces the bioadhesive from the container to the surgical site. A control switch, that is mounted on the housing, may operate the motorized actuator. The motorized actuator may be adapted to connect to the same electrical energy sources as the electrode or an independent electrical source.

These and other objects will be more clearly illustrated below by the description of the drawings and the detailed description of the various embodiments.

### BRIEF DESCRIPTION OF THE DRAWINGS

The accompanying drawings, which are incorporated in and constitute a part of this specification, illustrate embodiments of the present disclosure and, together with a general description of the disclosure given above, and the detailed description of the embodiments given below, serve to explain the principles of the present disclosure.
FIG. 1 is a perspective view of one embodiment of a surgical coagulator in accordance with the present disclosure showing a bioadhesive material applicator assembly (in phantom) disposed within a housing of the surgical coagulator;
FIG. 2A is a perspective view of the bioadhesive material applicator assembly of FIG. 1;
FIG. 2B is a perspective view of an alternate embodiment of a bioadhesive material applicator assembly;
FIG. 3A is a side view of a surgical coagulator showing an alternate embodiment of a bioadhesive applicator assembly in accordance with the present disclosure wherein the applicator assembly is disposed outside the housing of the surgical coagulator;
FIG. 3B is a side view of a surgical coagulator showing an alternate embodiment of a bioadhesive applicator assembly in accordance with the present disclosure having a syringe-like actuating pump for dispelling the bioadhesive to the tissue site; and
FIG. 3C is a side view of a surgical coagulator showing an alternate embodiment of a bioadhesive applicator assembly in accordance with the present disclosure having a motorized screw-like pump for dispelling the bioadhesive to the tissue site.

### DETAILED DESCRIPTION

Embodiments of the presently disclosed electrosurgical instrument are described in detail with reference to the drawing figures wherein like reference numerals identify similar or identical elements. As used herein, the term "distal" refers to that portion of the instrument, which is further from the user, while the term "proximal" refers to that portion of the instrument, which is closer to the user.

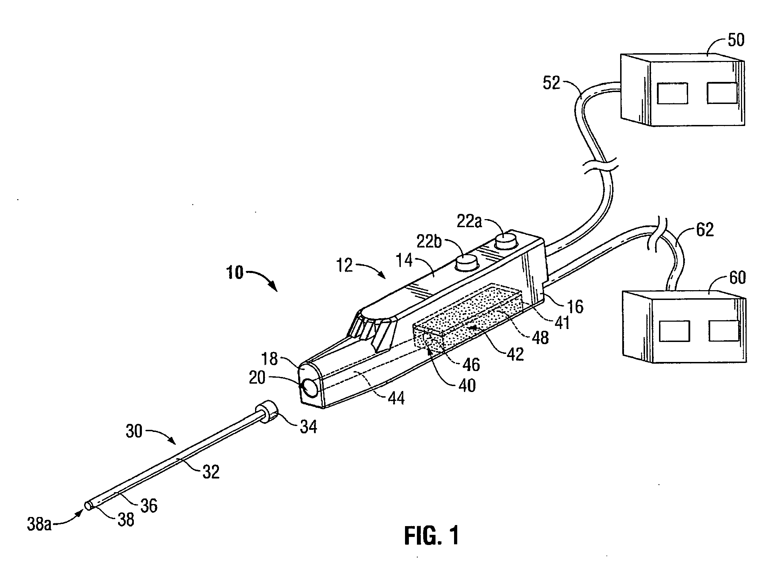

FIG. 1 sets forth a perspective view of an electrosurgical coagulator according to the present disclosure and is depicted generally as 10. The electrosurgical coagulator 10 includes a housing 12 having a handle 14 and proximal and distal ends 16 and 18, respectively. An elongated suction tube electrode 30 is fluidly and electrically coupled to a port or opening 20 defined in the distal end 18 of the housing 12 and extends therefrom. Suction tube electrode 30 may be selectively engageable with housing 12 or integrally formed therewith depending upon a particular purpose.

The suction tube electrode 30 includes an elongated tubular hollow shaft 32 having proximal and distal ends 34 and 36, respectively that may, for example, be constructed from a conductive metal that is partially covered by an insulative material to prevent electrical continuity along shaft 32. The distal end 36 is exposed to include a blunt electrode 38 that is configured and dimensioned to perform various electrosurgical coagulation procedures (e.g., tonsillectomy, adenoidectomy, etc.). The electrode 38 of the distal end 36 may be substantially blunt, rounded or include a pattern of protuberances to facilitate coagulation of tissue at or adjacent the distal end 36 when activated by the user. Suction tube electrode 30 is configured to electrically interface via the hollow shaft 32 to an electrosurgical generator 50 via one or more cables 52.

In embodiments, the generator 50 may control the amount of electrosurgical energy delivered to the tissue based on one or more electrical parameters via one or more sensors coupled to a feedback circuit. For example, the generator 50 may regulate, measure, monitor and/or control one or more of the following electrical or electromechanical parameters: electrical intensity, voltage, current, pulse rate, waveform, temperature and/or impedance. A return pad (not shown) may be utilized to complete the electrical circuit through the patient and the generator 50 may be configured to include patient return pad monitoring such as the system commonly sold under the trademark REM™ by Valleylab, Inc., of Boulder, Colorado.

Suction tube electrode 30 includes an aspiration port 38a defined through the distal end 36 of suction tube electrode 30. Aspiration port 38a is configured to facilitate the removal of surgical fluids and debris from the surgical site. In embodiments, the aspiration port 38a may be disposed through a side of suction tube electrode (not shown). More particularly and as shown in FIG. 1, the suction tube electrode 30 is connected in fluid communication to a source of negative pressure, i.e., vacuum 60, which draws air and fluid into the aspiration port 38a and into the vacuum via hose or tube 62 upon activation by the user. Aspiration port 38a may be chamfered, beveled or some other advantageous shape to create a smooth fluid stream therethrough and into the suction tube electrode 30 to facilitate fluid or debris removal. Moreover, suction tube electrode 30 may be made from a flexible and/or malleable material to give the user additional control of the coagulator 10 during use.

Housing 12 of the electrosurgical coagulator 10 also includes one or more control switches 22a and 22b which regulate the electrosurgical energy to the suction tube electrode 30. Either one of the control switches, 22a or 22b, disposed on the housing 12 may be utilized to control coagulation of the instrument 10, while the other control switch may be utilized to control suction of the instrument 10. In embodiments, a rotating or sliding-type switch may be employed to accomplish this purpose. Moreover, a switch regulator or potentiometer (e.g., a voltage divider network - VDN) may be used to vary the electrosurgical energy and/or the relative suction through tube 30.

Coagulator 10 includes a bioadhesive applicator assembly 40 operatively associated with the coagulator 10. Bioadhesive applicator assembly 40 generally includes a bladder or housing 41, which defines a reservoir 42 for containing a medicinal fluid 48 (e.g., a bioadhesive material). Reservoir 42 is disposed in fluid communication with a delivery lumen 44 defined between the bladder 41 and the suction tube electrode 30. Fluid 48 is defined herein to include fluids and gels that are suitable for or compatible with coagulation surgical procedures (e.g., prior to, during or after application of electrical energy). Some examples of medicinal fluids include bioadhesive fluids and gels which are biomaterial surgical sealants and adhesion barriers developed by hydrogel technology focused on adhesion prevention, tissue sealing and hemostatic clinical application, such as gels sold under the trademarks DURASEAL®, SPRAYGEL® and MICROMYST™, manufactured by CONFLUENT® Surgical, Inc. of Waltham, MA (a wholly owned subsidiary of U.S. Surgical, a Tyco Healthcare Company). Other bioadhesive examples include hemostatic matrices such as FLOSEAL™ manufactured by Baxter International, Inc. and SURGIFLO™. manufactured by Johnson & Johnson.

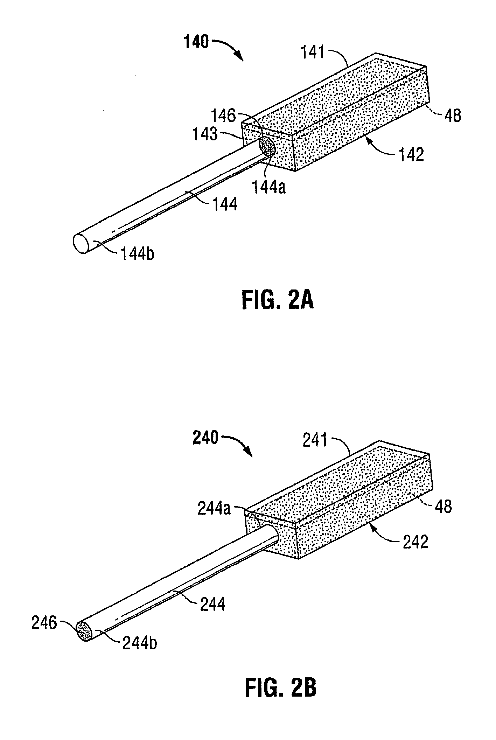

Turning now to FIGS. 2A and 2B, alternative embodiments of the present disclosure are illustrated. The delivery lumen 144, which includes proximal and distal ends, 144a and 144b, respectively, is attached in fluid communication with a distal end 143 of the reservoir 142 such that the delivery lumen 144 and the port 120 (not shown) align. A seal or plug 146 is disposed between the port 120 and the lumen 144 to allow selective expulsion of fluid 48 from reservoir 142 and for preventing fluid 48 from prematurely escaping from the reservoir 142. Seal 146 is disposed on the proximal end 144a of delivery lumen 144 in FIG. 2A, while seal 246 may be disposed on the distal end 244b of delivery lumen 244. In embodiments, seal 46, 146 and 246 may be disposed in any suitable location within bioadhesive applicator assembly 40, 140 and 240, respectively, such that fluid 48 is contained for selective application within the respective reservoir 42, 142 and 242. When pressure is applied by the user, the seal (e.g., seal 46) is configured to either break or open to force the bioadhesive material 48 from reservoir 42. Seal 46 may be a valve, to allow the user to selectively control the expulsion of fluid from reservoir 42 (e.g., duck bill valve, iris valve, etc.).

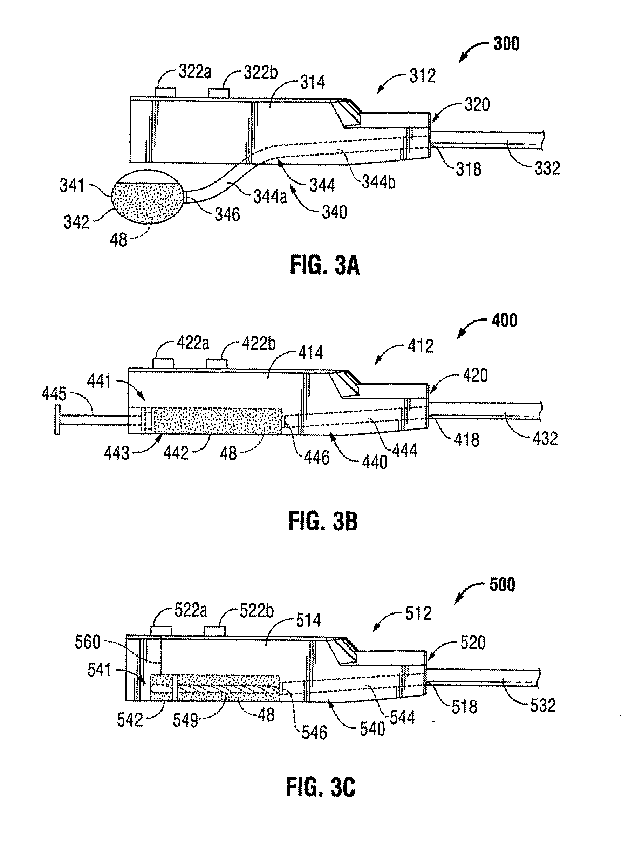

As seen in FIGS. 3B-3D, alternative embodiments of electrosurgical coagulator 10 are shown generally as 300, 400 and 500. The electrosurgical coagulators 300, 400, and 500 are similar to the coagulator 10 and will only be discussed in detail to the extent necessary to identify differences in construction and operation.

Electrosurgical coagulator 300 includes housing 312 and suction tube electrode 332 that is attached on the distal end 318 of the housing 312. Suction tube electrode 332 fluidly and electrically couples to housing 312 in a similar fashion as described above, with reference to coagulator 10. An external fluid applicator assembly 340, that includes a container, well or bladder 341 defining a reservoir 342 for containing fluid 48 is operatively attached to housing 312 via delivery lumen 344. The delivery lumen 344 has external and internal segments, 344a and 344b, respectively. When the user manually squeezes (e.g., applies pressure) the external fluid applicator assembly 340 (e.g., a squeezable bulb), the fluid 48 contained within reservoir 342 is expelled through the external and internal delivery lumen, 344a and 344b, respectively, into the suction tube electrode 332 to the surgical site. A seal 346 is included, which ruptures or regulates the flow of fluid 48 from reservoir 342.

FIG. 3B shows an alternate embodiment of a coagulator 400 according to the present disclosure wherein a bioadhesive applicator assembly 440 is disposed within the housing 412 of suction coagulator 400. The bioadhesive applicator assembly 440 includes a container 443 having a reservoir 442 with a syringe-like or plunger-like actuator 441 for dispensing the fluid 48 from reservoir 442. More particularly, the plunger-like actuator 441 includes a plunger head 445 that is configured and dimensioned to slidably fit within reservoir 442. The plunger head 445 is selectively movable from a first configuration wherein the fluid 48 is maintained within reservoir 442 to subsequent positions wherein incremental amounts of fluid 48 are dispensed through lumen 444 and tube 432 to the surgical site. In embodiments, the syringe reservoir 442 may be prepackaged with a particular medicinal fluid 48 and then inserted within the housing 412, either by the user or the manufacturer. In embodiments, the user manually operates the plunger head 445 to force the fluid 48 to break and/or open seal 446 similar to the seals described above.

FIG. 3C shows yet another embodiment of a coagulator 500 according to the present disclosure, which includes a motorized actuator for dispensing fluid 48 to the surgical site. More particularly, the bioadhesive applicator assembly 540 includes a motorized pump or actuator 541 that forces the fluid 48 from reservoir 542 through lumen 544, into suction tube electrode 532 and to the surgical site. Any suitable motorized pump may be used to drive the fluid 48 to suction tube electrode 532. Actuator 541 may be powered by the generator 50 (FIG. 1) with one or more controllers or buttons 522a, 522b attached therebetween or to the housing 512. A control wire or connector 560 may connect the button, e.g., 522a, to the actuator 541. The motorized applicator assembly 540 may alternatively be separately powered (e.g., battery powered). As shown in FIG. 3D, a screw-like actuator 541 rotates a screw gear 549 that drives fluid 48 from the reservoir 542 through the delivery lumen 544 and a seal 546. The fluid 48 then flows through the delivery lumen 544 through the suction tube electrode 532 and to the surgical site. The user can selectively regulate the amount of fluid 48 dispensed by controlling the screw 549 or the seal 546 or combinations thereof.

From the foregoing and with reference to the various figure drawings, those skilled in the art will appreciate that certain modifications can also be made to the present disclosure without departing from the scope of the present disclosure. For example, in embodiments, the coagulator may be manufactured such that the coagulator is disposable, reusable or reposable. Also in embodiments, a variety of different or interchangeable suction tube electrodes could be selectively attached to the distal end of the coagulator housing depending upon a particular purpose or to meet a particular surgical need. Additionally, in other embodiments, the suction coagulator, the electrode, and the fluid applicator assembly may be manually or remotely operated by the user by either a footswitch, or as mentioned above, a controller disposed on the instrument.

Referring back to FIG. 1, the suction coagulator 10 is shown having an internal compressible reservoir 42 disposed within the housing 12. As mentioned above, reservoir 42 may be disposed in any suitable location within the housing 12. The bottom portion of the housing 12 may be rubberized and integrated with the reservoir 42, so that when the user manually applies pressure to the reservoir 42, the bioadhesive fluid 48 is forced out the length of the delivery lumen 44, through the suction tube electrode 32 and out the aspiration port 38a for application to the surgical site.

In addition and although not shown, one or more of the actuators described herein on the bladder shown in FIG. 3B may be configured to provide a small amount of negative pressure (e.g., take in fluid) when release to limit unintentional leakage of additional fluid 48 to the surgical site.

Although the generator and vacuum are depicted as separate elements in FIG. 1, a vacuum may be included with the generator in particular embodiments.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of embodiments. Those skilled in the art will envision other modifications within the scope and spirit of the claims appended hereto.

## Claims

1. An electrosurgical suction coagulator, comprising:
a housing;
an elongated electrode having distal and proximal ends and adapted to connect to an energy source, the proximal end configured to operably couple to a distal end of the housing, the distal end configured to apply energy to tissue, the elongated electrode including a lumen defined therethrough that is operably coupled to a vacuum source; and
a fluid applicator assembly operably coupled to the elongated electrode and including a container defining a reservoir configured to contain a bioadhesive therein, wherein the bioadhesive is selectively dispensable from the container to deliver the bioadhesive to a surgical site.

2. The suction coagulator according to claim 1, wherein the fluid applicator assembly includes at least one of a seal and valve to selectively regulate the flow of bioadhesive to the surgical site.

3. The suction coagulator according to claim 1 or 2, wherein the fluid applicator assembly includes a bladder that is compressible to expel the bioadhesive from the container to the surgical site.

4. The suction coagulator according to claim 1 or 2, wherein the fluid applicator assembly includes a container that is compressible to expel the bioadhesive from the container to the surgical site.

5. The suction coagulator according to claim 4, wherein the fluid applicator assembly includes an actuator that is moveable from a first position wherein the bioadhesive is maintained in the container to at least one subsequent position to incrementally dispense the bioadhesive to the surgical site.

6. The suction coagulator according to claim 5, wherein the actuator includes a plunger.

7. The suction coagulator according to claim 5 or 6, wherein the actuator is motorized.

8. The suction coagulator according to claim 7, wherein the actuator includes at least one screw-like element that forces the bioadhesive from the container.

9. The suction coagulator according to claim 7 or 8, wherein the housing includes at least one control switch that operates the motorized actuator.

10. The suction coagulator according to claim 7, 8 or 9, wherein the motorized actuator is adapted to connect to the same electrical energy sources as the electrode.
